# EUROPEAN PATENT SPECIFICATION

(11) **EP 0 793 945 B1**
(45) Date of publication and mention of the grant of the patent: **19.07.2006**
(21) Application number: 97301549.8
(22) Date of filing: 07.03.1997
(51) Int. Cl.: A61B 17/17

(54) **Surgical positioning apparatus**
Chirurgische Positioniervorrichtung
Appareil chirurgical de positionnement

(30) Priority: 08.03.1996 GB 9604992
(43) Date of publication of application: 10.09.1997
(73) Proprietor: The University of Hull, Kingston-upon-Hull HU6 7RX (GB); Mohsen, Amr M.M.A., East Yorkshire HU14 3RG (GB); Sherman, Kevin P., North Ferriby, East Yorkshire HU14 3AW (GB); Karpinski, Marek R.K., Kirkella, East Yorkshire HU10 7TL (GB); Cain, Terence J., Skidby, East Yorkshire HU16 0EL (GB)
(72) Inventor: Phillips, Roger, North Ferriby, East Yorkshire HU14 3AT (GB); Viant, Warren J., Brough, East Yorkshire HU15 2TZ (GB); Griffiths John G., Cottingham, East Yorkshire HU16 5RQ (GB); Dyer, Kevin D.F., North Lincolnshire DN18 6AX (GB); Mohsen, Amr M.M.A., Swanland, East Yorkshire HU14 3RG (GB); Sherman, Kevin P., North Ferriby, East Yorkshire HU14 3AW (GB); Karpinski, Marek R.K., Kirkella, East Yorkshire HU10 7TL (GB); Cain, Terence J., Skidby, East Yorkshire HU16 0EL (GB)
(74) Representative: Hartley, Andrew Philip

(56) References cited:
- EP-A- 0 456 103
- WO-A-95/07055
- DE-C- 4 225 112
- DE-U- 8 806 671
- US-A- 4 750 487
- PROCEEDINGS OF THE INSTITUTION OF MECHANICAL ENGINEERS JOURNAL OF ENGINEERING IN MEDICINE. PART H, vol. 208, no. H02, PART H, 1 January 1994, XP000479710 POTAMIANOS P ET AL: "A ROBOTIC SYSTEM FOR MINIMAL ACCESS SURGERY"
- CLINICAL BIOMECHANICS, vol. 10, no. 6, 1 September 1995, pages 293-303, XP000515642 NOLTE L - P ET AL: "CLINICAL EVALUATION OF A SYSTEM FOR PRECISION ENHANCEMENT IN SPINE SURGERY"

## Description

The invention relates to an apparatus for positioning an implement relative to a feature. For example, the implement may be a surgical implement and the feature may be an anatomical feature of a patient or a surgical implant within a patient.

Many surgical procedures involve the positioning of a surgical implement with a precise spatial disposition relative to anatomical and/or surgical implant features. An example is the positioning of a drill for drilling a hole accurately along a path within a bone.

One such procedure involves drilling into a bone to produce a hole for receiving a screw. At present, the surgeon views a sequence of one or more real-time x-ray images of the bone taken intra-operatively (i.e. x-ray images taken in the operating theatre during the operation) from different angles. The surgeon estimates the spatial orientation of a drill required to produce a hole in a specific direction through the bone. Once drilling has begun, subsequent intra-operative x-ray images may be viewed to confirm the correctness of the path of the drill. Finally, the position of the pin within the bone may be confirmed by viewing additional x-ray images.

This type of procedure is necessarily time consuming, and it exposes the patient and nearby members of the surgical team to x-ray radiation. Also, if the screw is inaccurately placed, the treatment might not be fully effective and might fail, and the patient might need further treatment as a result.

According to a first aspect of the invention, there is provided an apparatus for positioning an implement relative to a feature comprising an x-ray unit having an x-ray detector and an x-ray source for directing x-rays towards the detector to produce at the detector an image of the feature representing variation in x-ray intensity at the detector in two mutually perpendicular directions lying in a plane transverse to the path of the x-rays between the source and the detector, the source and the detector being locatable in a first position relative to a datum to produce at the detector a first said image of the feature and being locatable at a second position relative to the datum to produce at the detector a second said image of the feature, means for producing information representing a position of the implement relative to the datum, and a processor connected to the x-ray unit for passage of the first and second images to the processor and connected to the implement position information producing means for passage of the implement position information to the processor, the processor being programmed to use the first and second images, information relating to the first position and the second position and the implement position information to produce information for positioning the implement in a required position relative to the feature.

Such an apparatus is disclosed in the paper : 'A robotic system for minimal access surgery' by P. Potamianos et al., Proceedings of the Institute of Mechanical Engineers Journal of Engineering in Medicine, Part H, vol. 208, no. H₂, 1.1.94 and is characterised in that the processor is adapted for applying a different respective correction of distortion to each x-ray image, each said correction being based on a comparison of relative positions of x-ray opaque or transparent areas in the corresponding image with the actual known positions of the areas.

The apparatus of the invention facilitates the accurate positioning of a surgical implement. Furthermore, use of the apparatus may result in a considerable reduction in exposure of the patient and nearby surgical team to x-ray radiation, compared with current practices. Additionally considerable reductions in the operation time may be achievable.

The following is a more detailed description of an embodiment of the invention, by way of example. Reference is made to the accompanying drawings in which:
Figure 1 is a diagram of some of the components of a surgical implement positioning apparatus;
Figure 2 is a diagrammatic perspective view of a locator board for use with the components of Figure 1;
Figure 3 is a diagram of a hollow steel rod located inside a femur bone;
Figure 4 is a diagrammatic perspective view of a part of a broken femur, and
Figure 5 is a diagrammatic view of the x-ray image cast by part of the hollow steel rod of Figure 3.

### Description of Apparatus

The surgical implement positioning apparatus comprises an x-ray unit 10, a mechanical arm 12, two infra-red cameras 14, a computer 16, a locator board 18, and a distortion detector board (not shown). The components of the apparatus are, in use, accommodated in an operating theatre.

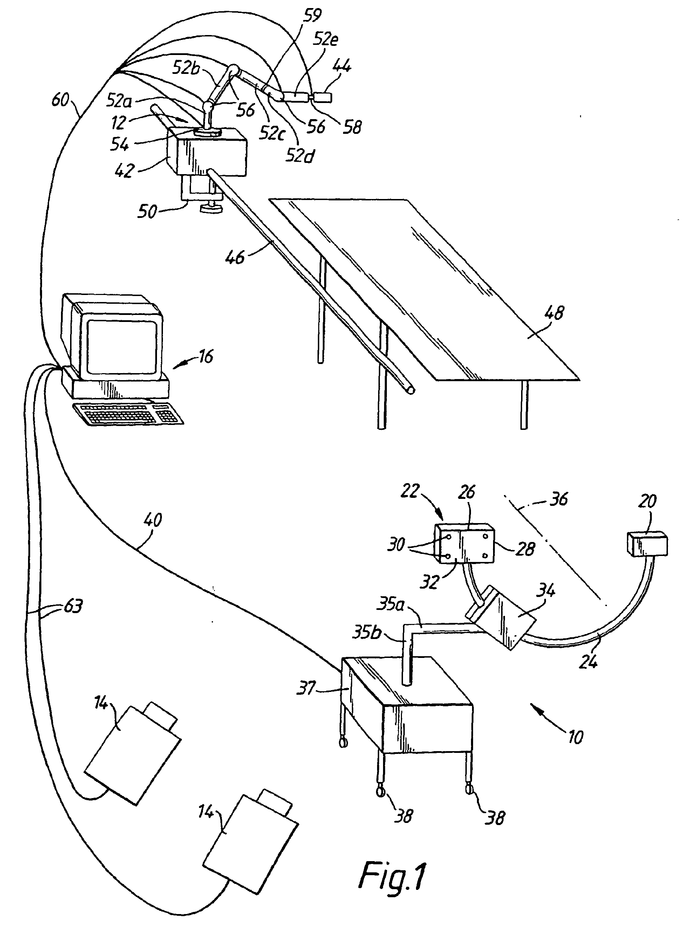

The x-ray unit 10 can be of any suitable type. The unit 10 described here is by way of example only. As shown in Figure 1, the x-ray unit 10 comprises a single x-ray source 20 and a single x-ray detector 22 secured at opposite ends of a semicircular support 24.

The x-ray detector 22 comprises a casing 26 which houses a thin detector sheet of ionisable material (not shown). One side of the sheet faces the source 20 and the other side is provided with a matrix of charge detector elements. In use, x-rays are directed from the source towards the detector and x-rays striking the sheet interact with the ionisable material to produce localised charges. Individual charges are detected by individual detector elements. Signals from the detector elements are amplified and passed to processing means within the x-ray unit 10 which process them to produce an image. Hence, an image is produced at the detector - the image representing variation in x-ray intensity at the detector in two mutually perpendicular directions lying in a plane transverse to the path of the x-rays between the source and the detector. The image is optionally displayed on a display monitor of the x-ray unit 10 and an electronically encoded form of the image is made available to the computer 16 via a cable 40.

The casing 26 includes a wall 28 which is transparent to x-rays and which lies between the detector sheet and the source 20. The wall 28 has an external surface which lies approximately parallel to the detector sheet. Four x-ray opaque spheres (not shown) are glued to the external surface of the wall 28 in a quadrilateral, nearly square, arrangement for a purpose to be described below. The arrangement is such that each sphere is visible on an x-ray image unless hidden by a dense object.

A side surface 32 of the casing 26, lying in a plane approximately normal to the wall 28, is provided with four infra-red emitters 30 arranged in a rectangular grouping. The positional relationship between the emitters 30 and the external surface of the x-ray transparent wall 28 is determined by a method described below, and data describing this relationship is then stored in the computer 16. Alternatively, any number exceeding two of emitters 30 may be mounted in any suitable arrangement on the casing 26.

The source 20 is of known type and, in use, generates x-rays at an origin within the source 20, which is treated as a point. x-rays travel from this point to the detector sheet as a diverging beam.

Although the x-rays are detected by the detector sheet, the computer 16 produces an image (as described in more detail below) as though the x-rays were detected at the outer surface of the transparent wall 28. The outer surface is termed the 'virtual detector sheet'. The computer is able to do this as the precise spatial relationship between the detector sheet and the virtual detector sheet is known and the approximate position of the x-ray origin relative to the virtual detector sheet is also known.

The semicircular support 24 passes through a mounting block 34 through which the support 24 can be moved so as to rotate the source 20 and the detector 22 in a plane perpendicular to the axis 36 of the arc described by the support 24. The mounting block 34 is mounted on a horizontal support member 35a which is connected to a vertical support member 35b which, in turn, is mounted to a base 37 of the unit 10. The lengths of the members 35a and 35b are adjustable. The mounting block 34 is attached to the horizontal member 35a with a rotary joint such that the semicircular support 24 can be rotated about the horizontal axis of the member 35a. This arrangement allows the source 20 and the detector 22 to be positioned in any orientation relative to the base 37.

The base 37 is provided with castors 38 for moving the X-ray unit 10 about the operating theatre. The castors 38 are lockable to stop the x-ray unit 10 from moving. The x-ray unit is provided with a panel with controls for taking x-ray images and controls for positioning some of the components of the x-ray unit (not shown).

The mechanical arm 12 has a base 42 connected to a guide 44 by five elongate members 52a-e. The guide 44 is adapted for holding a drill bit (not shown), such that the drill bit can be moved along a line with a precise position relative to the guide 44. The base 42 is mounted on a fixed horizontal support bar 46 extending along a lateral edge of an operating theatre table 48 and can be fixed in a required position along the bar 46 by a clamp 50. Alternatively, the base 42 may be mounted on any suitable support such that the guide can be manoeuvred into the space where it is needed for any particular operation. One alternative arrangement is to mount the base 42 onto the x-ray unit 10.

A first elongate member 52a projects upwards from the base 42, and is connected to the base 42 by a mounting 54 which allows rotation of the first member 52a relative to the base 42 around a generally vertical axis coinciding with the longitudinal axis of the first member 52a. The upper end of the first member 52a is connected to a first end of a second elongate member 52b by a joint 56 which permits pivotal movement of the second member 52b relative to the first member 52a around a generally horizontal axis normal to the first and second members 52a and 52b. The second end of the second member 52b is connected to a first end of a third member 52c by a joint 56 similar to the joint 56 connecting the first and second members 52a and 52b. The second end of the third member 52c is connected to a first end of a fourth member 52d by a joint 59 which allows the fourth member 52d to rotate relative to the third member 52c around an axis which coincides with the longitudinal axes of the third and fourth members. The second end of the fourth member 52d is connected to a first end of a fifth member 52e by a joint 56 similar to the joints 56 connecting the first and second members 52a,52b and second and third members 52b,52c. The guide 44 is connected to the second end of the fifth member 52e by a mounting 58 allowing rotation of the guide 44 relative to the fifth member 52e about an axis coinciding with the longitudinal axis of the fifth member 52e.

Thus the members 52a-e allow the guide 44 to be moved into any orientation relative to the base 42.

For each degree of freedom on a joint 56,59 there is provided a position sensor (not shown) of suitable type. The position sensor (or position sensors, where more than one is provided) produce a signal corresponding to the position of one of the associated members 52 relative to the other associated member. One suitable type of position sensor is based on optical encoding. Each optical encoder produces a digital signal which is transmitted to the computer 16 by a cable 60. Each joint 56,59 is also provided with a brake suitable for locking the joint 56,59 to prevent relative movement between the associated members 52. One such brake type operates electromagnetically. These brakes are connected to a suitable switch (not shown) which allows the brakes to be applied and released as required during the positioning of the drill guide 44.

Mountings 54 and 58 are similarly provided with position sensors connected to the computer 16. One of the sensors produces signals corresponding to the position of the first member 52a relative to the base 42, and the other sensor provides signals corresponding to the position of the guide 44 relative to the fourth member 52d. The mountings 54 and 58 are also provided with brakes connected to the switch, for locking the mountings 54 and 58 to prevent rotation between the associated components.

Six infra-red emitters (not shown) are mounted on the surface of the guide 44 for a purpose to be described below. Four of the emitters are grouped in a rectangle and the other two emitters lie in a plane orthogonal to the plane of the rectangular grouping. Alternatively, any number exceeding two of emitters may be mounted in any suitable arrangement on the guide 44.

In Figure 1, the portions of the cable 60 running from the joints 56,59 and the mounting 58 are shown outside the mechanical arm 12 for clarity. In practice, each member 52a-e is hollow, and the cable 60 can run inside the members 52a-e.

In use, the members 52a-e and the guide 44 may be covered by a sterilizable sleeve.

The computer 16 is of suitable type and comprises, for example, an 80486 micro-processor, a display monitor, a keyboard and a pointing device.

The infra-red cameras 14 are of suitable known type and operate in conjunction with the infra-red emitters 30 on the casing 26, the emitters on the guide 44 and also with intrared emitters (described below) on the locator board 18. The cameras 14 are fixed and separated from each other by a short distance in the operating theatre. The cameras 14 detect infra-red radiation from each emitter and generate signals for the determination of the spatial positions of the emitters. The positions of the emitters are determined with respect to a coordinate system fixed with respect to the cameras 14. The computer 16 uses the spatial positions of the emitters and the aforesaid stored information which represents the positions of the emitters 30 relative to the virtual detector sheet together with stored data which represents the positions of the emitters on the guide 44 to determine the spatial positions (with respect to the coordinate system) of the virtual detector sheet and the guide. Each camera 14 is connected to the computer by a cable 63.

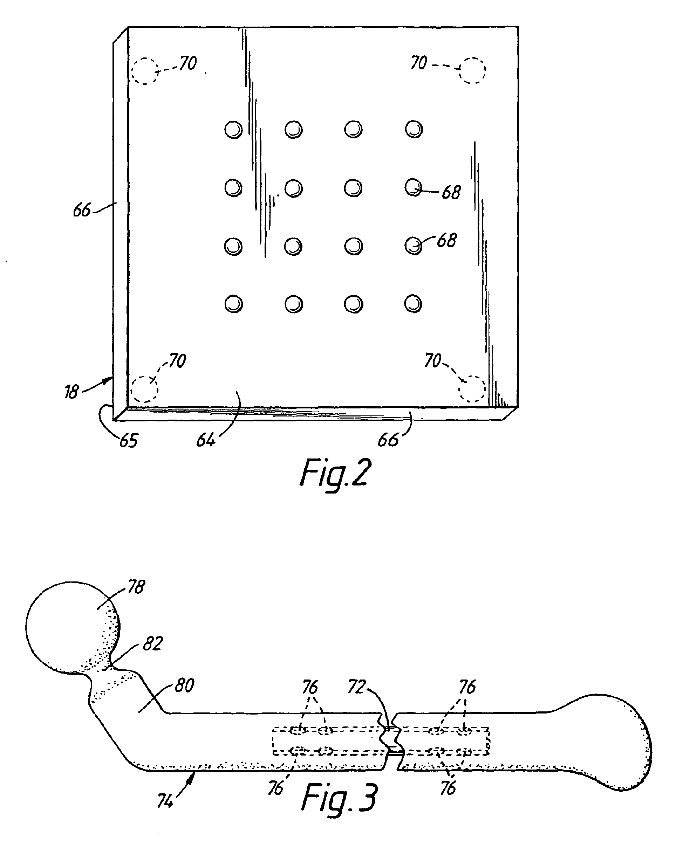

The locator board 18, shown in Figure 2, is precisely manufactured from a thick sheet of rigid material which is transparent to x-rays. The locator board is square in shape. Sixteen spheres 68, which are opaque to x-rays, are mounted on one side surface 64 of the board 18 in a four-by-four square matrix arrangement. The position of each sphere on the locator board 18 is known. Alternatively, any number, exceeding two, of spheres 68 may be mounted on the surface 64 so long as the positions of the spheres 68 on the board 18 are known.

Four infra-red emitters 70 are mounted at the corners of the side surface 65 of the board 18 which is opposite to the surface 64. The emitters 70 are precisely positioned relative to the spheres 68. Alternatively, any number, exceeding two, of emitters 70 may be mounted on the surface 65, in any suitable arrangement, so long as the precise positions of the emitters 70 relative to the spheres 68 are known.

The distortion detector board (not shown) is similar to the locator board 18 except that it is provided with nine hundred spheres 68, precisely positioned in a 30-by-30 square matrix on one side surface of the board. Also the board has no infra-red emitters 70. It is not essential that the spheres 68 are arranged in a 30-by-30 square matrix. Other arrangements comprising different numbers of spheres 68 may be used. Preferably, however, the first detector board has more spheres 68 than the locator board.

The apparatus described above is suitable for use during many surgical procedures, to assist in the precise positioning of a surgical implement relative to a feature of a patient, such as, for example, a bone or an implant, and for guiding the implement along a path relative to the feature. Although the general operation of the apparatus will be similar for most surgical procedures, the precise sequence of operations performed with the apparatus and the processing steps performed by the computer 16 are tailored to suit individual surgical procedures.

The operation of the apparatus is described below firstly for the positioning and guiding of a drill during the repair of a broken femur. A hole needs to be drilled along a very precise path through the femur so that the drill bit passes through an existing pair of holes in a surgical implant inside the hollow of the bone. The x-ray unit 10 is used, in combination with the cameras 14 and the computer 16, to determine the precise spatial position of the path along which the drill bit must be guided in order to achieve this. This is done by taking x-ray images of the femur from different directions. For each image, key points or lines in the image are identified and the precise spatial positions of the lines or planes extending from the x-ray origin to the virtual detector sheet, and corresponding to the key points or lines are determined. The spatial position of the implant and the path for the drill bit are calculated from the positions of these lines or planes.

However, before the position of the path for the drill bit is determined, several processes are preferably performed with the apparatus to maximize the accuracy of determination of the position of the path. Firstly, the distortion detector board is used to detect and correct for distortion in the x-ray images. These images are distorted because of the design of the x-ray unit and by variations in the magnetic field surrounding the x-ray unit.

### Detection of and Correction for Distortion in the x-Ray Images

The distortion detector board is clamped to the wall 28 of the x-ray unit 10. This board is placed so that the surface containing the spheres 68 is against the x-ray transparent wall 28. Thus, the steel spheres 68, fixed in the board, lie in the path of x-rays travelling between the source 20 and the detector sheet. The area of the matrix of spheres 68 and the position of the board are such that the image of the board on the detector sheet covers the whole detector sheet. The x-ray unit 10 is then operated to produce an image of the spheres. The computer 16 then analyses this image to determine the spacing of the spheres in the image, and compares this with their known real spacing. The distortion is not so bad that it is not possible to match a sphere with its x-ray image. The spheres are arranged in a square grid pattern. A square defined by 4 spheres at its corners appears as a distorted quadrilateral on the x-ray image. A mathematical function (including bilinear interpolation) is constructed which maps every pixel in the interior of each quadrilateral onto its correct location within the interior of the corresponding square on the distortion detector board, thus correcting the distortion. The mathematical pixel mappings constructed in this way are used to correct for distortion in all subsequent images.

However, the distortion present in an image depends on the position of the x-ray unit when the x-ray image was produced. Thus, the computer correction of distortion as described above only removes distortion completely for a given position of the x-ray unit 10. Further correction is required for images produced after the x-ray unit has been re-positioned. This further correction is performed using the four spheres glued to the wall 28, as described below.

### Determination of the Positional Relationship between the Virtual Detector Sheet and the Emitters 30

The next step, before the x-ray unit 10 is repositioned, is to use the locator board 18 to determine the precise positional relationship between the virtual detector sheet and the emitters 30 on the casing 26.

The first step is to clamp the locator board 18 to the wall 28 of the x-ray unit 10; i.e. against the virtual detector sheet.

The board 18 is placed so that the surface 64 containing the spheres 68 lies against the wall 28 and the surface 65 containing the infra-red emitters 70 faces away from this wall 28.

The cameras 14 then generate signals which are passed to the computer 16. These signals give the positions of the emitters 70 on the locator board 18 and the positions of the emitters 30 attached to the side surface 32 of the casing 26. Simultaneously, the x-ray unit 10 is operated to produce an image of the spheres 68 on the locator board 18.

As the precise dimensions of the locator board 18 are known, the computer 16 can calculate, from the positions of the emitters 70, the position of the plane containing the virtual detector sheet (which, as indicated above, now abuts the locator board 18). Also, the precise positions of the spheres 68 can be calculated from the positions of the emitters 70. Knowing the positions and dimensions of the spheres 68, the computer 16 can calculate the positions of the notional images of the spheres on the virtual detector sheet (in other words, the circular regions on the virtual detector sheet which are shielded from x-rays by the spheres 68) and relate these positions to the locations of the images of the spheres 68 in the electronic image analysed by the computer 16.

Hence, the position of the virtual detector sheet is determined and related to the positions of the emitters 30 on the casing 26. As indicated above, data describing the positional relationship between the virtual detector sheet and the emitters 30 is stored in the computer 16. This data can be used to determine, from any given positions of the emitters 30, the notional position on the virtual detector sheet of any part of an image analysed by the computer 16.

### Determination of the Positional Relationship between the x-ray Origin and the Emitters 30

The next step, also before the x-ray unit 10 is repositioned, is to use the locator board 18 to determine the precise positional relationship between the x-ray origin and the emitters 30.

Firstly the board 18 is positioned approximately parallel to the virtual detector sheet and approximately mid-way between the source 20 and the virtual detector sheet. The x-ray source 20 is then operated to produce an image of the spheres 68. At the same time, the cameras 14 co-operate with the infra-red emitters 70 on the board 18 to produce signals relating to the position of the board 18, and with the emitters 30 on the casing 26 to produce signals relating to the position of the virtual detector sheet. The computer 16 then calculates the positions of the notional images of the spheres 68 on the virtual detector sheet. This is done by first locating the image of the spheres on the x-ray image. The spheres being dense have a high contrast level and can be easily identified, unless obscured by another dense object. Once the location of the sphere has been found, a mathematical algorithm analyses the change in contrast level over the sphere's image. Using this information, the algorithm is able to accurately determine the precise position of the notional image of the sphere on the virtual detector sheet.

Thus the computer 16 has information which defines the positions in space of the virtual detector sheet, the board 18, the notional images of the spheres 68 on the virtual detector sheet (corrected for distortion), and data defining the actual positions of the spheres 68 (as the positions of the spheres 68 relative to the emitters 70 are known). From this information, the computer determines the positions of the lines connecting each sphere 68 with its associated image and projects these lines backwards to determine the position of the x-ray origin.

Once this is done, data reflecting the relative positioning of the origin and the infra red emitters 30 is stored in the computer 16. This data can be used to determine, for any given positions of the emitters 30, the precise position in space of the x-ray origin. Hence, for any given positions of the emitters 30, the computer 16 can determine the position of a line connecting the x-ray origin to a point on the virtual detector sheet corresponding to a given point in an electronic image.

### Further Correction for Distortion in x-ray Images taken after Repositioning the x-ray Unit 10

Before the x-ray unit 10 is repositioned, the unit is operated to produce a reference x-ray image showing only the spheres glued to the x-ray transparent wall 28. The computer 16 then analyses this x-ray image (after correction for distortion as described above) to determine the position of the spheres in the image. As previously stated, repositioning the x-ray unit 10 introduces some distortion into an x-ray image taken at the new position. This is due mainly to the change in position of the x-ray unit in relation to the Earth's magnetic field but may also be caused by flexing of the support 24 due to gravity.

The spheres glued to the wall 28 remain attached to the wall 28 throughout an operation. When the x-ray unit 10 is operated during an operation, images of these spheres appear in the x-ray image unless hidden by dense objects. The computer 16 analyses each image (after correction for distortion as described above) to determine the position of the spheres in the image. The computer then compares the new position of each sphere visible in the x-ray image with the corresponding position of the sphere from the reference x-ray image. From these positional changes, the computer determines a further correction for the x-ray image so as to remove the distortion that results from the change in position of the x-ray unit 10. This is achieved in the following way. Any x-ray picture taken, following repositioning of the x-ray unit 10, contains an image of the spheres attached to the wall 28. The picture is distorted and thus the arrangement of spheres in the image is also distorted. The distortion is not so bad as to prevent the determination of which image sphere corresponds to which particular sphere attached to the transparent wall 28. The distorted arrangement of sphere images is then mapped mathematically onto the reference image, thus correcting the distortion using a process similar to that described above in respect of the distortion detector board.

### Determination of Drilling Path through Femur

With information in the computer 16 to correct for image distortion, and for calculating the spatial positions of the x-ray origin and the virtual detector sheet from the positions of the emitters 30, the apparatus can be used to determine the position of the path for the drill bit through the femur. This is done as follows, with reference to Figure 3.

A broken femur 74 (Figure 3) has its upper part connected to the pelvis by a ball-joint (the ball or head is shown at 78) and its lower part connected to the knee. A hollow steel rod 72 of generally circular cross section is introduced by the surgeon into the intra medullary canal of the femur 74 and extends along its length, bridging the break. The rod 72 is to be locked to both the lower and upper parts of the femur 74, and for this purpose each end of the rod 72 has two pairs of diametrically opposed holes 76. The fixing of each end of the rod 72 requires two holes to be drilled through the femur 74, so that each hole is aligned with a respective one of the pairs of holes 76 at that end of the rod 72. Respective screws are then inserted into each hole and through the corresponding pair of holes 76.

The insertion of locking screws for the upper end of the rod 72 is straightforward. One approach to locating these screws involves attaching a jig to the upper end of the rod 72. The insertion of locking screws for the lower end of the rod 72 is made more difficult because the shape of the rod 72 distorts as it is inserted into the femur 74. The following describes a method for the insertion of these locking screws.

For each pair of rod holes 76, the apparatus operates to determine the position of the axis that passes through the centres of these two holes, and then uses this information to aid the surgeon to position the drill-guide 44 in the appropriate position for guiding the drill bit into the femur 74 along the determined axis. This is achieved as follows.

A patient with a broken femur is placed on the operating table 48 and the patient's leg being operated upon is immobilised in a generally horizontal position. The x-ray unit 10 is then brought to the operating table 48 and positioned for taking, in a generally vertical direction, an image of the portion of the femur 74 containing the rod 72. The x-ray unit 10 is fixed in this position. The x-ray unit 10 is then operated to produce an anterior-posterior (AP) x-ray image of the femur 74, and at the same time, the cameras 14 are operated to locate the emitters 30 for calculation of the positions of the virtual detector sheet and the origin.

The x-ray unit 10 is then repositioned for taking another image of the portion of the femur 74 containing the rod 72 such that the direction of the x-ray beam is generally horizontal. The x-ray unit 10 is fixed in this position. The x-ray unit 10 is then operated to produce a lateral x-ray image of the femur 74. At the same time the cameras 14 are again operated to detect the positions of the emitters 30.

The computer 16 analyses both the lateral and AP x-ray images. Each image is formed by areas of lightness and darkness. The generally parallel side edges of the rod 72, when imaged side-on by the unit 10, will appear in the image as very distinct generally parallel lines at the interface between a dark area (corresponding to the rod) and a surrounding relatively light area. The computer 16 is programmed to scan each image and locate these two lines in the image. Alternatively, the position of these lines can be selected using a pointing device from a displayed image. The computer then determines the spatial position of each of the corresponding notional lines on the virtual detector sheet and, knowing the spatial position of the x-ray origin, calculates, for each line, the spatial position of the plane including the notional line and the origin. These planes lie tangentially to the rod 72.

The spatial position of the rod 72 is calculated by the computer 16 from the intersections of the tangential planes determined as described above.

The lateral image is also scanned by the computer 16 to find the edges of the images of the pairs of holes 76 in the rod 72. The shapes of the images depend on the position and orientation of the rod 72 relative to the virtual detector sheet. If, by chance, the axis through a pair of holes 76 coincides with the line joining the x-ray origin to the centre of the virtual detector sheet, the image of the pair of holes will appear as a perfect circle at the centre of the virtual detector sheet.

Traditionally, the surgeon seeks this axis by manipulating the x-ray unit 10 until the image of a hole is as nearly circular and at the centre of the screen as the surgeon can judge by eye. If, from this position, the x-ray beam was gradually rotated about the axis of the rod 72 in a plane perpendicular to the axis of the rod 72, the image of the hole would gradually change shape. It would consist of the intersection of two ellipses. The points of intersection of these two ellipses would appear to lie on the axis of the rod 72. Since the new shape is determined by the angle theta through which the x-ray beam is turned, it follows that theta can be found from a knowledge of the new shape. Suitable characteristics of the image are measured for this purpose. These characteristics have been carefully selected to minimise the destabilising effects of unavoidable uncertainty about the accuracy of the shape of the image. If, after rotating, the beam were to be translated parallel to itself, the effect would be to shift the image away from the centre of the screen. Hence, by working backwards from any image, it is possible to move the image intensifier to obtain a perfect circle at the centre of the screen. There is no need to do this, unless for checking purposes. It is necessary only to place the drill along what would be the new x-ray axis.

This information is then used to position the guide 44. This is done as follows.

### Using the Mechanical Arm to Position a Drill Bit

The mechanical arm 12 is placed next to the femur 74. The base 42 is then clamped in a suitable position. The cameras 14 co-operate with the infra-red emitters on the guide 44 to provide the computer 16 with information relating to the position of the guide 44. The position sensors (not shown) provided at the joints 56,59 and the mountings 54,58 of the arm 12 provide the computer 16 with information which defines the position of the base 42 relative to the guide 44. A drill bit (not shown) is held in the guide 44 and the computer 16 includes information regarding the position of the drill bit in the guide 44.

The guide 44 is then moved towards a position in which the drill bit is aligned with the drilling axis for one of the pairs of holes 76 in the rod 72. The computer 16 monitors this movement using data from the position sensors and provides an indication of the convergence of the actual position of the drill bit with its optimal position. An experienced surgeon will know approximately the optimal position of the drill, so large deviations from the optimal position are unlikely to occur.

The guide 44 is moved until the computer indicates that the drill bit is aligned with the drilling axis for one of the pairs of holes. The joints 56,59 and the mountings 54,58 are then locked by the brakes to prevent further movement of the members 52a-e. The drill bit is then drilled along the axis through the bone on one side of the femur 24, through the pair of holes 76 and into the bone on the other side of the femur. During this procedure the drill bit is guided along the axis by the guide 42. A screw is then inserted to secure the rod 72 within the femur 76. This above process is repeated for the second pair of holes 76.

The information stored in the computer 16 may also be used to position the x-ray unit 10 such that the pair of holes 76 gives a circular image. This can provide confirmation to the surgeon that the apparatus has correctly analysed the data or assist the surgeon in manoeuvring the x-ray unit to a better position. The computer 16 monitors such movements of the x-ray unit, using data obtained from the emitters 30 and the cameras 14, and the computer provides an indication of the convergence of the actual position of the x-ray unit to the desired position of the x-ray unit.

### Use of the Positioning Apparatus for Positioning a Drill during the Repair of a Broken Femur Neck

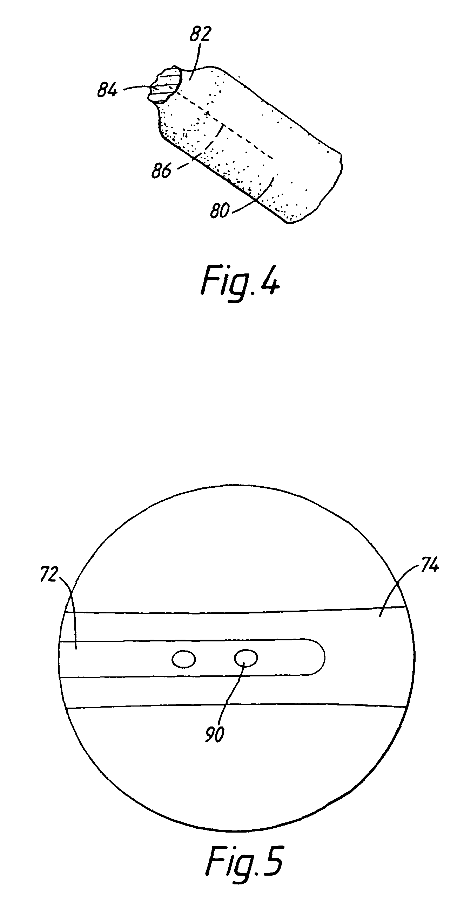

The apparatus described above (with reference to the drawings) can also be used in other procedures. As an example, the following describes a method for the insertion of a pin into a femur 74 to repair a fracture in the neck 82 of a femur 74. As shown in Figures 3 and 4, the neck 82 of a femur 74 is a narrow portion connecting the femur head 78 to an angled end portion 80 of the femur 74. In this procedure, a hole is drilled along the axis of the angled end 80, through the neck 82 and into the head 78 and the pin is inserted into the hole. Plainly, the hole needs to be properly aligned so that it passes close to the centres of these parts. The method to be described can also be used for the fixation of other devices for the repair of fractures to the upper end of the femur.

The current procedure for repairing a fractured femur neck 82 involves a surgeon viewing x-ray images of these parts 78,80,82 of the femur 74 and deciding on a path for drilling. A guide hole is drilled and a thin metal guide rod is inserted. The position of the rod is viewed on an x-ray image. If the rod is not correctly aligned, then without removing the rod, a further guide hole is drilled and another guide rod inserted, and a further image is viewed. Drilling and viewing guide holes is repeated until a guide hole is drilled which appears to be correct. All guide rods except the correctly positioned rod, are removed. The correct hole is enlarged and the pin inserted around the guide rod. Finally the guide rod is removed.

This process is lengthy and depends on the eye of the surgeon for accuracy. The use of many x-ray images involves a significant exposure to x-rays. If the hole for the pin is incorrectly positioned the pin may break out of the head 78 of the femur 74 when a force is applied thus preventing a successful outcome from the operation. The apparatus described here (with reference to the drawings) overcomes these difficulties as follows.

First, the distortion detector board, the locator board 18 and the spheres glued to the wall 28 are used in the preparatory procedures described above to provide image correction data and to determine the precise relative positions of the x-ray origin, the virtual detector sheet and the emitters 30. The patient is then brought to the operating table 48 and the femur 74 is immobilised in a roughly horizontal position. The x-ray unit 10 is then brought to the operating table 48 and positioned horizontally for taking, generally in side elevation, an image of the upper part of the femur including the head 78 and neck 82. The x-ray unit 10 is fixed in this position. The x-ray unit 10 is then operated to produce an image of the end of the femur 74, and, at the same time, the cameras 14 are operated to detect the positions of the emitters 30. The computer 16 produces a distortion-free image of the femur 74.

The image is then analysed to produce information about features of the femur 74 that allow the required drilling path to be determined- There are several features that can be used, but the present description is based on a requirement that the drilling path pass approximately through the centre of the neck 82 and into the centre of the head 78.

To allow the computer 16 to determine the position of the drilling path the computer 16 needs to determine firstly the position of the centre of the neck 82 and secondly the position of the centre of the head 78. These are both determined by analysing the image produced with the x-ray unit 10 in the horizontal position described above, and analysing a second image produced with the x-ray unit 10 rotated through 90°, into a generally vertical disposition.

The position of the centre of the neck 82 is determined as follows. Firstly, the horizontal image is analysed to identify the two generally arcuate lines in the image corresponding to the "side edges" of the neck 82 seen in side elevation. Secondly, two points, one on each of the arcuate lines, which are the mutually closest such point are identified. Thirdly, the centre point of the line joining the mutually closest points is determined. Finally, the position of the line joining the x-ray origin to the point on the virtual detector sheet corresponding to the above mentioned centre point is calculated. The same procedure is performed with the vertical image, thereby giving the position of a second line between the origin and the virtual detector sheet (the respective positions of the origin and the virtual detector sheet being different for the first and second such lines).

If the two lines joining the origin and the virtual detector sheet intersect, the point of intersection is taken to be the centre of the neck 82. If these lines do not intersect, the centre of the neck 82 is taken to be the midpoint of the shortest line joining the two lines.

Finding the position of the centre of the head 78 is achieved by finding the position, in each image, of a line marking the outer surface of the head 78. This can be done either by computer analysis of the image, or, since the algorithms for such analysis may be complex, by the surgeon interacting with the computer 16. One suitable means of interaction involves a mouse and pointer with the mouse being "clicked" when the pointer is at spaced points along this arcuate line. An alternative suitable means would be a touch sensitive screen where the surgeon draws in the outline of the head.

For each image, the centre point of the arcuate line bounding the head is calculated, and the position of a line joining the x-ray origin to the point on the virtual detector sheet corresponding to the centre point is determined. If the two lines joining the origin and the virtual detector sheet intersect, the point of intersection is taken to be the centre of the head 78. If these lines do not intersect, the centre of the head 78 is taken to be the midpoint of the shortest line joining the two lines.

The computer 16 thus has in store the position of the centre of the head and the position of the centre of the neck and the computer 16 can construct a line joining the two and passing along the angled end 80. This is the required drilling path. The above description is by way of an example for determining a drilling path into the femur head 78. Additional features of the femur may be considered in determining the required drilling line, for example, the longitudinal axis of the femur and preoperative x-ray images.

This information is then used to position the guide 44 as described above with reference to the first procedure. The hole is then drilled through the neck and head of the femur and the pin 72 inserted.

The apparatus described above (with reference to the figures) is not limited to the two procedures described. There are many other procedures in which the apparatus may be useful. Some of these may involve drilling (for example drilling holes in the spinal region) while others may involve the alignment of other surgical instruments (for examples saw and puncturing tools) to perform medical procedures. As will be clear from the above, the method is particularly useful for positioning the implement (the drill in the above examples) relative to hard tissue, such as bone, which strongly blocks the passage of x-rays.

The apparatus need not be exactly as described above. For example, the locator board 18 and the distortion detector board, or their equivalents, could be used, in whole or in part, in maintenance procedures and/or the manufacturing process for the x-ray unit. Also the locator board 18 and the distortion detector board (or their equivalents), could be integral components of the x-ray unit.

The use of the cameras 14 may be a problem where persons or equipment within the operating theatre obstruct the line of sight between a camera and an emitter. For this reason, more than two cameras 14 may be provided and these may be spaced around the operating theatre and /or above the operating table. This may have the additional advantage of providing a more accurate position signal. The positions of the infra-red emitters and the cameras could be reversed; cameras could be provided on the x-ray unit 10 and the mechanical arm 12 and infra-red emitters provided around the operating theatre.

The purpose of x-ray opaque spheres on the locator board 18, the distortion detector board and the x-ray transparent wall 28 is to provide a single point of reference on an x-ray image - the centre of the sphere. Therefore, a sphere could be replaced by another object opaque to x-rays, such as a small cross or disc. Furthermore, there are other configurations of spheres, other than those described above, which can provide the required points of reference in an image. Alternatively, the locator board 18 could be constructed from a square of x-ray opaque material (instead of x-ray translucent material as described above) with holes drilled in the board providing the reference points in the image (instead of x-ray opaque spheres providing the reference points as described above).

The locator board 18, the distortion detector board and the spheres glued to the wall 28 serve different purposes. However, they may be combined together. For example, a board could be devised which combines the purpose of the locator board 18 and the distortion detector board.

The locator board 18 could also be present in every x-ray image during the operation. By analysing the images of the spheres 68 and the information gained from the cameras 14 about the sensors 70 it is possible for a mathematical algorithm to determine the position of the virtual detector sheet. This would remove the need for sensors 30.

Although the apparatus displays to the surgeon an image from the detector sheet of the x-ray unit, it need not produce such a display. It could produce a purely electronic image which is analysed by the computer 16.

The mechanical arm 12 is provided principally to provide the surgeon with a means to position the surgical implement accurately. It could, however, be replaced by an arm with some motorised components (under the control of the computer) that move the implement automatically to the required position. An alternative approach would be not to have an arm 12 and, instead, have some means of tracking position attached to the surgical tool with the computer 16 giving guidance to the surgeon to obtain the required tool position.

The determination of the path for the surgical implement need not be determined during the operation. The path could be determined before the operation using any suitable imaging device, and this path used by the computer 16 during the operation.

The apparatus described above has uses other than assisting a surgeon to perform a surgical procedure. For example, it may be used to train surgeons to position an implement, or guide an implement along a course, relative to an anatomical, or implant feature. In this case the apparatus may be used in conjunction with artificial body parts.

## Claims

1. Apparatus for positioning an implement relative to a feature comprising an x-ray unit (10) having an x-ray detector (22) and an x-ray source (20) for directing x-rays towards the detector (22) to produce at the detector (22) an image of the feature representing variation in x-ray intensity at the detector (22) in two mutually perpendicular directions lying in a plane transverse to the path of the x-rays between the source (20) and the detector (22), the source (20) and the detector (22) being locatable in a first position relative to a datum to produce at the detector (22) a first said image of the feature and being locatable at a second position relative to the datum to produce at the detector (22) a second said image of the feature, means (12,14) for producing information representing a position of the implement relative to the datum, and a processor (16) connected to the x-ray unit (10) for passage of the first and second images to the processor and connected to the implement position information producing means (12, 14) for passage of the implement position information to the processor (16), the processor (16) being programmed to use the first and second images, information relating to the first position and the second position and the implement position information to produce information for positioning the implement in a required position relative to the feature, **characterised in that** the processor (16) is adapted for applying a different respective correction of distortion to each x-ray image, each said correction being based on a comparison of relative positions of x-ray opaque or transparent areas in the corresponding image with the actual known positions of the areas.

2. Apparatus according to claim 1, wherein the x-ray unit (10) is mobile relative to the datum with a working area, the apparatus including means (14, 30) for producing information representing a position of the source (20) and the detector (22) relative to the datum at any position of the unit (10) with the working area, the processor (16) using said source and detector position information to produce said information for positioning the implement.

3. Apparatus according to claim 2, wherein the means (14, 30) for producing said source and detector position information comprises a plurality of emitters (30) and a plurality of receivers (14) for receiving, without requiring any physical connection to the emitters (30), signals emitted from the emitters (30), one of the pluralities being fixed relative to the source (20) or the detector (22) and the other one of the pluralities being fixed relative to the datum.

4. Apparatus according to claim 3, wherein the emitters are infra-red emitters (30) and the receivers are cameras (14).

5. Apparatus according to claim 3 or claim 4, wherein the emitters (30) are fixed relative to the source (20) or the detector (22) and the receivers (14) are fixed relative to the datum.

6. Apparatus according to any preceding claim, wherein the means (12,14) for producing the implement position information comprises a holding means (12) having an implement holding portion (44) moveable with respect to and connected to a securing portion (42) for securing the holding means (12) to a support (46).

7. Apparatus according to claim 6, wherein the holding means (12) includes sensor means for producing information representing the position of the holding portion (44) relative to the securing portion (42).

8. Apparatus according to claim 6 or claim 7, when claim 6 is dependent on claim 3, wherein the means (12,14) for producing the implement position information also comprises the plurality (14) fixed relative to the datum and a further plurality of emitters or receivers, as the case may be, fixed to the holding means (12) for interacting with the plurality (14) fixed relative to the datum to produce information representing the position of the holding means (12) relative to the datum.

9. Apparatus according to any one of claims 1 to 8, wherein the processor (16) is programmed to analyse the images to identify a part of the feature therein.

10. Apparatus according to any one of claims 1 to 9, wherein the processor (16) includes a pointing device for selecting a part of the feature in the images.

11. Apparatus according to any preceding claim, wherein the processor (16) generates information for positioning the implement on a required course for the implement relative to the feature, the required position lying on the course.

12. Apparatus according to any preceding claim, wherein the processor (16) generates information representing the required position relative to the datum.

13. Apparatus according to any one of claims 1 to 12, including a board having areas relatively opaque to x-rays and areas relatively transparent to x-rays for compensating for distortion in the images.

14. Apparatus according to claim 5 or any claim dependent thereon, including a board (18) having areas (68) relatively opaque to x-rays and areas relatively transparent to x-rays, said board also being provided with a plurality of emitters (70).

## Patentansprüche

1. Vorrichtung zum Positionieren eines Gerätes in Bezug auf ein Merkmal, welche eine Röntgeneinheit (10) mit einem Röntgendetektor (22) und einer Röntgenquelle (20) zum Aussenden von Röntgenstrahlen auf den Detektor (22), um an dem Detektor (22) eine Abbildung des Merkmals zu erzeugen, das eine Veränderung in der Röntgenintensität an dem Detektor (22) in zwei wechselseitig senkrechten Richtungen, die einer Ebene quer zu dem Pfad der Röntgenstrahlen zwischen der Quelle (20) und dem Detektor (22) liegen, repräsentiert, wobei die Quelle (20) und der Detektor (22) in einer ersten Position in Bezug auf einen Festpunkt positionierbar sind, um an dem Detektor (22) eine erste Abbildung des Merkmals zu erzeugen, und an einer zweiten Position in Bezug auf den Festpunkt positionierbar ist, um bei dem Detektor (22) eine zweite Abbildung des Merkmals zu erzeugen; eine Einrichtung (12, 14) zum Erzeugen von Information, die eine Position des Gerätes in Bezug auf den Festpunkt repräsentiert; und einen Prozessor (16) aufweist, der mit der Röntgeneinheit (10) zur Weitergabe der ersten und zweiten Abbildungen an den Prozessor verbunden ist und mit der Gerätepositions-Informationserzeugungseinrichtung (12, 14) zur Weitergabe der Gerätepositionsinformation an den Prozessor (16) verbunden ist, wobei der Prozessor (16) dafür programmiert ist, die ersten und zweiten Abbildungen, die Information bezüglich der ersten Position und der zweiten Position und die Gerätepositionsinformation zu nutzen, um Information zur Positionierung des Gerätes in einer erforderlichen Position in Bezug auf das Merkmals zu erzeugen, **dadurch gekennzeichnet, dass** der Prozessor (16) dafür eingerichtet ist, eine unterschiedliche entsprechende Verzerrungskorrektur auf jedes Röntgenbild anzuwenden, wobei jede Korrektur auf einem Vergleich von Relativpositionen von röntgenundurchlässigen oder -transparenten Bereichen in dem entsprechenden Bild mit den tatsächlich bekannten Positionen der Bereiche basiert.

2. Vorrichtung nach Anspruch 1, wobei die Röntgeneinheit (10) in Bezug auf den Festpunkt in einem Arbeitsbereich beweglich ist, wobei die Vorrichtung eine Einrichtung (14, 30) zum Erzeugen von Information enthält, die eine Position der Quelle (20) und des Detektors (22) in Bezug auf den Festpunkt bei jeder Position der Einheit (10) in dem Arbeitsbereich repräsentiert, und wobei der Prozessor (16) die Quellen- und Detektor-Positionsinformation nutzt, um die Information für die Positionierung des Gerätes zu erzeugen.

3. Vorrichtung nach Anspruch 1, wobei die Einrichtung (14, 30) zum Erzeugen der Quellen- und Detektor-Positionsinformation eine Vielzahl von Emittern (30) und eine Vielzahl von Empfängern (14) aufweist, um, ohne irgendeine physische Verbindung mit den Emittern (30) zu erfordern, von den Emittern (30) emittierte Signale zu empfangen, wobei eine von den Vielzahlen in Bezug auf die Quelle (20) oder den Detektor (22) fixiert ist, und die andere von den Vielzahlen in Bezug auf den Festpunkt fixiert ist.

4. Vorrichtung nach Anspruch 3, wobei die Emitter Infrarot-Emitter (30) und die Empfänger Kameras (14) sind.

5. Vorrichtung nach Anspruch 3 oder 4, wobei die Emitter (30) in Bezug auf die Quelle (20) oder den Detektor (22) fixiert sind, und die Empfänger (14) in Bezug auf den Festpunkt fixiert sind.

6. Vorrichtung nach einem der vorstehenden Ansprüche, wobei die Einrichtung (12, 14) zum Erzeugen der Gerätepositionsinformation eine Halteeinrichtung (12) mit einem Geräthalteabschnitt (44) enthält, der in Bezug zu einem Feststellungsabschnitt (42) beweglich und damit verbunden ist, um die Halteeinrichtung (12) an einem Träger (46) festzustellen.

7. Vorrichtung nach Anspruch 6, wobei die Halteeinrichtung (12) eine Sensoreinrichtung zum Erzeugen von Information enthält, die die Position des Halteabschnittes (44) in Bezug auf den Feststellungsabschnitt (42) repräsentiert.

8. Vorrichtung nach Anspruch 6 oder 7, wenn der Anspruch 6 vom Anspruch 3 abhängt, wobei die Einrichtung (12, 14) zum Erzeugen der Gerätepositionsinformation auch die Vielzahl (14), die in Bezug auf den Festpunkt fixiert ist, und eine weitere Vielzahl von Emittern oder Empfängern je nach Fall aufweist, die an der Halteeinrichtung (12) fixiert sind, um mit der Vielzahl (14) in Wechselwirkung zu treten, die in Bezug auf den Festpunkt fixiert sind, um Information zu erzeugen, die die Position der Halteeinrichtung (12) in Bezug auf den Festpunkt repräsentiert.

9. Vorrichtung nach einem der Ansprüche 1 bis 8, wobei der Prozessor (16) dafür programmiert ist, die Abbildungen zu analysieren, um einen Teil des Merkmals darin zu identifizieren.

10. Vorrichtung nach einem der Ansprüche 1 bis 9, wobei der Prozessor (16) eine Zeigevorrichtung enthält, um einen Teil des Merkmals in den Abbildungen auszuwählen.

11. Vorrichtung nach einem der vorstehenden Ansprüche, wobei der Prozessor (16) Information zum Positionieren des Gerätes auf einem erforderlichen Kurs für das Gerät in Bezug auf das Merkmals enthält, wobei die erforderliche Position auf dem Kurs liegt.

12. Vorrichtung nach einem der vorstehenden Ansprüche, wobei der Prozessor (16) Information erzeugt, die die erforderliche Position in Bezug auf den Festpunkt repräsentiert.

13. Vorrichtung nach einem der Ansprüche 1 bis 12, mit einer Platte mit Bereichen, die für Röntgenstrahlen relativ undurchlässig sind und Bereichen, die für Röntgenstrahlen relativ transparent sind, um eine Verzerrung in den Bildem zu kompensieren.

14. Vorrichtung nach Anspruch 5 oder einem davon abhängigen Anspruch, die eine Platte (18) mit Bereichen (68), die für Röntgenstrahlen relativ undurchlässig sind und Bereichen, die für Röntgenstrahlen relativ transparent sind, beinhaltet, wobei die Platte auch mit einer Vielzahl von Emittern (70) versehen ist.

## Revendications

1. Appareil destiné à positionner un instrument par rapport à un élément caractéristique comprenant une unité de rayons X (10) ayant un détecteur de rayons X (22) et une source de rayons X (20) pour diriger des rayons X vers le détecteur (22) afin de produire au niveau du détecteur (22) une image de l'élément caractéristique représentant une variation de l'intensité des rayons X au niveau du détecteur (22) dans deux directions mutuellement perpendiculaires se trouvant sur un plan transversale par rapport au trajet des rayons X entre la source (20) et le détecteur (22), la source (20) et le détecteur (22) pouvant être positionnés dans une première position par rapport à un repaire pour produire au niveau du détecteur (22) une dite première image de l'élément caractéristique et pouvant être positionnés au niveau d'une seconde position par rapport au repaire pour produire au niveau du détecteur (22) une dite seconde image de l'élément caractéristique, des moyens (12, 14) pour produire des informations représentant une position de l'instrument par rapport au repaire, et un processeur (16) connecté à l'unité de rayons X (10) pour le passage des première et seconde images dans le processeur et connecté aux moyens de production d'informations (12, 14) relatives à la position de l'instrument pour le passage des informations relatives à la position de l'instrument dans le processeur (16), le processeur (16) étant programmé pour utiliser les première et seconde images, des informations concernant la première position et la seconde position et les informations relatives à la position de l'instrument afin de produire des informations pour le positionnement de l'instrument dans une position nécessaire par rapport à l'élément caractéristique, **caractérisé en ce que** le processeur (16) est adapté pour appliquer une correction respective différente de déformation pour chaque image de rayons X, chacune desdites correction étant basée sur une comparaison des positions relatives des zones opaques ou transparentes de rayons X dans l'image correspondante avec les positions connues réelles des zones.

2. Appareil selon la revendication 1, dans lequel l'unité de rayons X (10) est mobile par rapport au repaire avec une zone utile, l'appareil comprenant un moyen (14, 30) destiné à produire des informations représentant une position de la source (20) et du détecteur (22) par rapport au repaire au niveau de toute position quelconque de l'unité (10) avec la zone utile, le processeur (16) utilisant lesdites informations relatives à la position de la source et du détecteur pour produire lesdites informations destinées au positionnement de l'instrument.

3. Appareil selon la revendication 2, dans lequel le moyen (14, 30) destiné à produire lesdites informations relatives à la position de la source et du détecteur comprend une pluralité d'émetteurs (30) et une pluralité de récepteurs (14) destinés à recevoir, sans nécessiter aucune connexion physique aux émetteurs (30), des signaux émis par les émetteurs (30), l'un parmi la pluralité d'émetteurs étant fixe par rapport à la source (20) ou au détecteur (22) et l'autre parmi la pluralité d'émetteurs étant fixe par rapport au repaire.

4. Appareil selon la revendication 3, dans lequel les émetteurs sont des émetteurs infrarouges (30) et les récepteurs sont des caméras (14).

5. Appareil selon la revendication 3 ou 4, dans le quel les émetteurs (30) sont fixes par rapport à la source (30) ou au détecteur (22) et les récepteurs (14) sont fixes par rapport au repaire.

6. Appareil selon l'une quelconque des revendications précédentes, dans lequel le moyen (12, 14) destiné à produire des informations relatives à la position de l'instrument comprend un moyen de maintien (12) ayant une partie de maintien (44) d'instrument pouvant être déplacé par rapport à une partie de fixation (42) et connectée à celle-ci pour fixer le moyen de maintien (12) à un support (46).

7. Appareil selon la revendication 6, dans lequel le moyen de maintien (12) comporte un moyen formant capteur pour produire des informations représentant la position de la partie de maintien (44) par rapport à la partie de fixation (42).

8. Appareil selon la revendication 6 ou 7, lorsque la revendication 6 dépend de la revendication 3, dans lequel le moyen (12, 14) destiné à produire des informations relatives à la position de l'instrument comprend également la pluralité (14) fixe par rapport au repaire et une pluralité supplémentaire d'émetteurs ou de récepteurs, selon le cas, fixés au moyen de maintien (12) pour interagir avec la pluralité (14) fixe par rapport au repaire pour produire des informations représentant la position du moyen de maintien (12) par rapport au repaire.

9. Appareil selon l'une quelconque des revendications 1 à 8, dans lequel le processeur (16) est programmé pour analyser les images afin d'y identifier une partie de l'élément caractéristique.

10. Appareil selon l'une quelconque des revendications 1 à 9, dans lequel le processeur (16) comprend un dispositif de pointage destiné à sélectionner une partie de l'élément caractéristique dans les images.

11. Appareil selon l'une quelconque des revendications précédentes, dans lequel le processeur (16) génère des informations destinées à positionner l'instrument sur une course nécessaire de l'instrument par rapport à l'élément caractéristique, la position nécessaire se trouvant sur la course.

12. Appareil selon l'une quelconque des revendications précédentes, dans lequel le processeur (16) génère des informations représentant la position nécessaire par rapport au repaire.

13. Appareil selon l'une quelconque des revendications 1 à 12, comprenant une planche ayant des zones relativement opaques aux rayons X et des zones relativement transparentes aux rayons X pour compenser une déformation dans les images.

14. Appareil selon la revendication 5 ou l'une quelconque des revendications qui dépend de celle-ci, comprenant une planche (18) ayant des zones (68) relativement opaques aux rayons X et des zones relativement transparentes aux rayons X, ladite planche étant également prévue avec une pluralité d'émetteurs (70).
